Europäisches Patentamt

European Patent Office

Office européen des brevets

(11) Publication number: **0 315 300**
**A2**

# EUROPEAN PATENT APPLICATION

(21) Application number: 88307548.3

(51) Int. Cl.⁴: **A61K 37/02**

(22) Date of filing: 15.08.88

The applicant has filed a statement in accordance with Rule 28 (4) EPC (issue of a sample only to an expert). Accession number(s) of the deposit(s): FERM BP-852, FERM P-8199.

(30) Priority: 05.11.87 JP 280677/87

(43) Date of publication of application:
10.05.89 Bulletin 89/19

(84) Designated Contracting States:
AT BE CH DE FR GB IT LI LU NL SE

(71) Applicant: Takeda Chemical Industries, Ltd.
27, Doshomachi 2-chome Higashi-ku
Osaka-shi Osaka, 541(JP)

(72) Inventor: Tsuyuguchi, Izuo
363-4, Oda-cho
Izumi Osaka 594(JP)
Inventor: Sunada, Yutaka 10- 302, 5 Shiroyama
1-chome
Toyonaka
Osaka 561(JP)
Inventor: Tsuchiya, Kanji
10-6, Chigusa 3-chome
Takarazuka Hyogo 665(JP)

(74) Representative: Laredo, Jack Joseph et al
Elkington and Fife Beacon House 113
Kingsway
London, WC2B 6PP(GB)

(54) Treatment of tuberculous and nontuberculous mycobacterial infection.

(57) An agent containing an interleukin-2 active substance is markedly effective in treatment of tuberculous and nontuberculous mycobacterial infection of a human patient.

EP 0 315 300 A2

# TREATMENT OF TUBERCULOUS AND NONTUBERCULOUS MYCOBACTERIAL INFECTION

The present invention relates to a treatment of tuberculous and nontuberculous mycobacterial infection with an agent containing an interleukin-2 active substance, and to the agent.

It is well known that tuberculous and nontuberculous mycobacterial infection is a time-honored disease entity. However, the advent of drugs for treating tuberculous and nontuberculous mycobacterial infection in the latter half of the 1940's and the use of them in conjunction with surgical intervention resulted in sharp declines in both mortality rate and prevalence rate in the opulent countries. This was followed by the advent of highly effective drugs for treating tuberculous and nontuberculous mycobacterial infection in the latter half of the 1970's, which resulted in nearly 100% negative conversion in sensitive cases and the duration of treatment was also drastically curtailed. While the number of patients with tuberculous and nontuberculous mycobacterial infection has thus been decreased, the disease has not been completely eradicated as yet. To account for this persistence, the following factors have been suggested.

1) Patient of primary tuberculous and nontuberculous mycobacterial infection is still encountered and the prevalence rate is not declining.

2) The frequency of infection is on the steady increase among the elderly and among patients having diabetes, blood diseases or malignant tumors as underlying diseases and being treated with anticancer drugs, adrenocorticoids or immunosuppressant drugs so that tuberculous and nontuberculous mycobacterial infection is presenting a picture of refractory disease.

3) There has been an increasing number of patients infected with anti-tuberculous and - nontuberculous mycobacterial infection drug-resistant strains of tuberculous mycobacteria or nontuberculous mycobacteria and of patients who cannot be adequately treated on account of side effects of the drugs.

The current standard chemotherapeutic regimen for pulmonary tuberculous and nontuberculous mycobacterial infection (fortified chemotherapy) based on the recommendation of the special therapeutic committee of The Japanese Society for Tuberculosis is a 6- to 9-month treatment course using isoniazid (INH) and rifampicin (RFP), as cardinal drugs, plus either streptomycin (SM) or ethambutol (EB). Indeed, if such a therapeutic program could be executed on a continuous basis for a sufficient time, almost all cases of infections would be successfully cured.

Actually, however, even the above-mentioned treatment program spares many refractory cases. A first refractory group consists of patients with primary infection due to resistant mutants of tuberculous or nontuberculous mycobacteria and if the strains involved are resistant to either INH or RFP, the therapeutic efficacy of the regimen is substantially undermined. A second group consists of patients in whom repeated administration is prevented by side effects of the drug or complications (such as chronic hepatitis, hepatic cirrhosis, etc.). A third group covers both the elderly and patients with certain underlying diseases (such as diabetes, blood diseases, malignant tumors, etc.) in whom the effects of the drugs cannot be fully implemented. While even cases of primary infection are sometimes refractory as described above, the mycobacteria involved in recurrent infection are usually multiple drugs-resistant so that long-term hospitalization is often inevitable.

Thus, the current chemotherapy is quite ineffective for such chronic active cases and there has been a standing need for a drug, in addition to chemotherapeutics, that will enhance the antiinfective competency of the host.

The present inventors tried a human interleukin-2 active substance in patients with refractory tuberculous and nontuberclous mycobacterial infection and found such therapeutic effects as disappearance of mycobacteria from secretions, liquidation of fever, improvement in erythrocyte sedimentation rate and so on. The present invention is predicated on the above findings.

The present invention is directed to: (1) A method for treating a human patient suffering from tuberculous and nontuberculous mycobacterial infection, which comprises administering an effective amount of an interleukin-2 active substance as the active ingredient to the patient; (2) A pharmaceutical composition for the treatment of tuberculous and nontuberculous mycobacterial infection of a human patient, which comprises an effective amount of an interleukin-2 active substance and a pharmaceutically acceptable vehicle, carrier or diluent therefor; and (3) Use of an interleukin-2 active substance for the production of a pharmaceutical preparation for treating a tuberculous and nontuberculous mycobacterial infection in a human patient.

In this specification, interleukin-2 is sometimes referred to briefly as IL-2.

Tuberculous and nontuberculous mycobacterial infection in the context of the invention covers all the clinical conditions induced by tuberculous mycobacteria or nontuberculous mycobacteria.

The IL-2-active substance mentioned above may be any substance having IL-2 activity, that is to say any substance that is capable of proliferation and activation of T-cells.

Thus, for example, naturally-occurring IL-2 produced in the animal body or animal cells and recombinant IL-2 and related substances obtained by genetic engineering technique can be mentioned. The IL-2 includes proteins which have sugar chains and proteins which has no sugar chain.

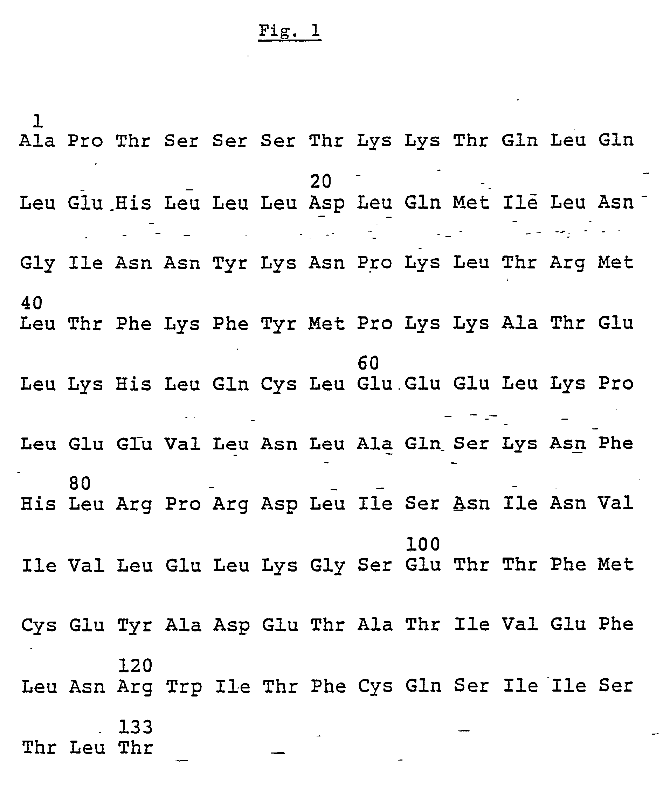

To cite a specific example, a recombinant polypeptide (I) prepared by genetic engineering technique and having an amino acid sequence shown in Fig. 1 (Japanese Patent Application Laid-open No. 78799/1986 which corresponds to EP Publication No. 176,299) or any fragment thereof that is essential to its biological or immunological competency can be employed in the practice of the invention. Examples of said fragment include a fragment which is available on elimination of one amino acid residue (EP Publication No. 91539) or 4 amino acid residues [Japanese Patent Application Laid-open No. 126088/1985] from the N-terminus of polypeptide (I) and a fragment available on elimination of a few amino acid residues from the C-terminus. Likewise useful is a fragment corresponding to the above-mentioned polypeptide (I) whose amino acid sequence is partly missing or has been partly replaced with one or more other amino acids, for example, one with the cysteine residue in 125-position replaced with a serine residue (Japanese Patent Application Laid-open No. 93093/1984 which corresponds to U.S. Patent No. 4,518,584).

The aforementioned recombinant IL-2 produced by genetic engineering technique may additionally have a methionine residue at the N-terminus of polypeptide (I) (Japanese Patent Application Laid-open No. 78799/1986 which corresponds to EP Publication No. 176,299 or may be a mixture of polypeptide (I) and polypeptide (I) additionally having a methionine residue at the N-terminus (Japanese Patent Application Laid-open No. 115528/1985 which corresponds to EP Publication No. 145,390).

Furthermore, the aforementioned IL-2 may have been chemically modified with a polyethylene glycol derivative, for instance (e.g. Japanese Patent Application Laid-open No. 226821/1985 which corresponds to EP Publication No. 154,316).

The specific activity of such IL-2 active sub stances is preferably about 10,000 to 50,000 units/mg and more desirably about 30,000 to 40,000 units/mg. The assay of IL-2 activity and the definition of IL-2 activity unit are as set forth in Japanese Patent Application Laid-open No. 115528/1985 which corresponds to EP Publication No. 145,390.

The IL-2 active substances that can be employed in accordance with the invention are low in toxicity. For example, the IL-2 produced by genetic engineering technique purified by the procedure described in Japanese Patent Application Laid-open No. 115528/1985 which corresponds to EP Publication No. 145,390 and isolated by the procedure described in Japanese Patent Application Laid-open No. 78799/1986 which corresponds to EP Publication No. 176,299, whose amino acid sequence is as shown in Fig. 1 and whose specific activity is about $3.5 \times 10^4$ units/mg, shows no lethal toxicity when it is administered to cynomolgus monkeys in a single intravenous dose of 6 mg/kg.

Since IL-2 active substances are, thus, low in toxicity, they can be safely administered to patients.

The agent for treating tuberculous and nontuberculous mycobacterial-infection according to the invention is preferably administered in the form of an injection.

Thus, the agent of the present invention can be made available as an aqueous solution or a solid preparation obtainable by freezing or lyophylizing the same.

If desired, the agent of the present invention containing an IL-2 active substance may be made available in the form of a mixture with a pharmaceutically acceptable diluent, vehicle, carrier or the like in accordance with the established pharmaceutical procedure.

To prepare an injectable aqueous solution, the agent of the present invention is formulated with, for example, a solvent such as water (e.g. distilled water), an aqueous vehicle (e.g. physiological saline, Ringer's solution), an oleagineus vehicle (e.g. sesame oil, olive oil), etc., if necessary together with solubilizers (e.g. sodium salicylate, sodium acetate), buffers (e.g. sodium citrate, glycerol), isotonizing agents (e.g. glucose, invert sugar), stabilizers (e.g. human serum albumin, polyethylene glycol), preservatives (e.g. benzyl alcohol, phenol), soothing agents (e.g. benzalkonium chloride, procaine hydrochloride) and other additives by the established pharmaceutical procedure.

The concentration of said IL-2 active substance in the above aqueous solution is about 1 to 10,000 µg/ml and preferably about 5 to 50 µg/ml. The concentration in activity unis (U) is about 35 to 350,000 U/ml and preferably about 175 to 1,750 U/ml.

This aqueous solution is preferably adjusted to pH about 3 to 7 and still more desirably to pH about 3.5 to 4.5. This adjustment is made, for example, by addition of diluted hydrochloric acid or dilute alkali solution (e.g. a dilute aqueous solution of sodium hydroxide or sodium hydrogen carbonate).

3

The agent according to the present invention can be formulated into a solid injectable preparation which is extemporaneously dissolved in a vehicle for parenteral administration. Such preparations can be manufactured by mixing the IL-2 active substance with, for example, a diluent (e.g. distilled water, physiological saline, aqueous glucose solution), an excipient (e.g. carboxymethylcellulose (CMC), sodium alginate), a preservative (e.g. benzyl alcohol, benzalkonium chloride, phenol), a soothing agent (e.g. glucose, calcium gluconate, procaine hydrochloride) and so on by the established pharmaceutical procedure.

Advantageously, human serum albumin (hereinafter sometimes referred to briefly as HSA) may be added to the agent of the invention and the solution be adjusted to pH about 3 to 7, for losses of IL-2 activity during storage and in the course of freezing and lyophylizing procedures will then be minimized and, in the case of a lyophylized preparation, the reconstituted solution will be clear.

HSA may be of any type. For example, it may be a HSA purified form healthy human plasma by Cohn's ethanol fractionation method (fraction No. 6).

The concentration of HSA in the aqueous solution of IL-2 active substance is preferably about 0.1 to 50 mg/ml and still more desirably about 0.5 to 20 mg/ml.

The present agent having immunomodulating actions does in many cases display beneficial effects when used in combination with other drugs for treating tuberculous and nontuberculous mycobacterial infection and the use of the agent of the present invention in such applications is also subsumed in the concept of the present invention.

Thus, enhanced efficacy may develop when the agent of the present invention is used in combination with other drugs for treating tuberculous and nontuberculous mycobacterial infection such as isoniazid, rifampicin, streptomycin, ethambutol, kanamycin, capreomycin, ethionamide, protionamide, enviomycin, pyrazinamide, paraaminosalicylate, cyclomolin, thioacetazone and so on.

The agent according to the present invention is preferably provided in the form of an aqueous solution, frozen preparation or lyophylized preparation. Particularly, a lyophylized preparation is appropriate.

For the purpose of preventing the attenuation of IL-2 activity, the agent of the present invention can be manufactured by the following procedures, for instance.

To an aqueous solution of IL-2 active substance, HSA is optionally added in the above-defined concentration range and the mixture is adjusted to an appropriate pH mentioned hereinbefore. If desired, an isotonizing agent, a surfactant (e.g. Tween 20, HCO-60) and so on may be further added as mentioned above. When such substances other than HSA are added, the pH of the final solution is adjusted to the aforesaid range by the procedure described before. The resulting aqueous solution can be used as a stock material in the preparation of the frozen or lyophylized preparation described below.

The frozen agent according to the present invention can be generally manufactured by freezing the above aqueous solution at about -80 to -20°C, for instance. The frozen preparation is preferably stored at about -80 to -20°C.

The lyophylized agent according to the present invention can be manufactured by drying the above frozen preparation under reduced pressure in the conventional manner or freezing the aforementioned aqueous solution or a reconstituted solution therefrom in small portions in the manner described as above and, then, subjecting the frozen solution to drying under reduced pressure in the conventional manner.

The frozen or lyophilized preparation manufactured in the above manner can be reconstituted with a vehicle, optionally adjusted to an appropriate pH with, for example, hydrochloric acid, to give a solution of the agent of the present invention.

To prepare the lyophilized agent of the present invention for parenteral use, it is preferable to blend an aqueous solution of IL-2 active substance with an aqueous solution of additives, both purified by passage through a bacterial filter, for instance, either before or after blending, distribute the mixture aseptically into dosage unit containers such as vials, and subject the filled dosage units to the aforementioned lyophilizing procedure. In this procedure, the stability of the agent of the present invention can be enhanced by evacuating the vial or purging with nitrogen gas.

For administration in the form of an injection, the agent of the present invention, when it is an aqueous solution, can be used as it is.

When the agent is a lyophilized solid preparation, it is reconstituted with distilled water, physiological saline or the like to give a solution for injection.

The agent of the present invention is markedly effective in the improvement or treatment of tuberculous and nontuberculous mycobacterial infection and is of value for the purpose.

The agent of the present invention can be administered to humans for treatment of tuberculous and nontuberculous mycobacterial infection caused by tuberculous mycobacteria or nontuberculous mycobacteria. The infections caused by tuberculous mycobacteria are, for example, pulmonary, articular, renal,

4

genitourinary, peritoneal, intestinal, pleuristic tuberculous mycobacterial infections.

The infections caused by nontuberculous mycobacteria are, for example, pulmonary, lymph node, bone, subcutaneous, skin, meninges, respiratory nontuberculous mycobacterial infections.

Microorganisms which may cause the tuberculous and nontuberculous mycobacterial infection referred to in this specification include, among others, tuberculous mycobacteria [for example, human type tuberculous mycobacteria (Mycobacterium tuberculosis), bovine type tuberculous mycobacteria (Mycobacterium bovis)], and nontuberculous mycobacteria (for example, Mycobacterium avium-complex (formerly designated as Mycobacterium avium-intracellulare), Mycobacterium kansasii, Mycobacterium scrofulaceum, Mycobacterium fortuitum).

For parenteral administration, the agent of the present invention can be injected intravenously, intramuscularly or subcutaneously, for instance. The proper dosage of IL-2 active substance depends on the route of administration and the severity of condition. Generally speaking, however, it can be administered to humans in a parenteral dose of about 1 to 7000 units/kg/day, preferably about 1 to 100 units/kg/day, and the range of 4 to 40 units/kg/day is particularly advantageous. It should be understood that the above range is merely illustrative because the optimum dosage is dictated by the method and duration of administration. The frequency of administration is preferably daily over a period of about 5 days to 3 months.

The agent of the present invention can be administered until complete cure is obtained or until such a stage where the response obtained so far is already sufficient to allow the immunopotency of the patient to take over to attain a cure.

The use of the agent of the invention leads to a marked improvement of various symptoms of tuberculous and nontuberculous mycobacterial infection or a cure of the disease without side effects and, therefore, is expected to contribute a great deal to practices in the departments of internal medicine and surgery where tuberculous and nontuberculous mycobacterial infection are treated.

In the specification and drawing where amino acids are designated by abbreviations, the abbrivations according to IUPAC-IUB Commission on Biochemical Nomenclature or those in routine use in this field of technology have been used as shown by the following examples. Where any amino acid involves optical isomers, the L-isomer is meant unless otherwise specified.

Gly : glycine

Ala : alanine

Val : valine

Leu : leucine

Ile : isoleucine

Ser : serine

Thr : threonine

Cys : cysteine

Met : methionine

Glu : glutamic acid

5

```
Asp       :  aspartic acid

Lys       :  lysine

Arg       :  arginine

His       :  histidine

Phe       :_ phenylalanine

Tyr       :  tyrosine

Trp       :  tryptophan

Pro       :  proline

Asn       :  asparagine

Gln       :  glutamine

Asp & Asn    :  aspartic acid and asparagine

Glu & Gln    :  glutamic acid and glutamine
```

Brief Description of the Drawing

Figure 1 shows the amino acid sequence of a representative human IL-2.

The following clinical case and working examples are further illustrative of the invention and should by no means limitative thereof, of course.

The IL-2 used in the following Clinical Examples and Examples is a human IL-2 having alanine at the N-terminus which is produced by cultivating a transformant Escherichia coli N4830/pTB285 (IFO-14437, FERM BP-852) by the method described in Japanese Patent Application Laid-open No. 78799/1986 which corresponds to EP Publication No. 176,299, purified to a high degree of purity by the method described in Japanese Patent Application Laid-open No. 115528/1985 which corresponds to EP Publication No. 145,390 and, then, isolated by the mutual separation procedure described in Japanese Patent Application Laid-open No. 78799/1986 which corresponds to EP Publication No. 176,299. Its amino acid sequence is as shown in Fig. 1 and its specific activity is about $3.5 \times 10^4$ U/mg. In this specification, one unit represents human IL-2 activity in 1 ml of an arbitrarily selected standard preparation and corresponds to about 28.6 ng of pure human recombinant IL-2.

The above-mentioned transformant Escherichia coli N4830/pTB285 has been deposited as of April 25, 1985 in the Institute for Fermentation, Osaka (IFO), Japan under the accession number of IFO 14437. It has also been deposited as of April 30, 1985 at Fermentation Research Institute, Agency of Industrial Science and Technology, the Ministry of International Trade and Industry (FRI), Japan under the accession number of FERM P-8199. This deposit has been converted to a deposit under the Budapest Treaty, with the microorganism being stored at FRI under the accession number of FERM BP-852.

Clinical Example 1

The patient, a 76-year-old woman, had a past history of pulmonary tuberculous mycobacterial infection which developed at the age of about 45 and was cured with antituberculosis drugs. This case is recurrent infection, yielding many nontuberculous mycobacteria showing a negative nyacin test (Gaffky 8). The isolated bacteria were identified to be Mycobacterium avium-complex and were completely resistant to rifampicin and almost resistant to ethambutol and isoniazid. The patient was treated by the administration of rifampicin 300 mg/day, ethambutol 0.5 g/day and isoniazid 300 mg/day through oral administration after admission in a hospital and continued for 5 months but no effect was achieved. Thereafter, concurrent intravenous administration of IL-2 in a dose of 1000 U/day was started as an adjunct to the above regimen. As a result, the bacteria ceased to be discharged and the patient became afebrile and showed improvement in erythrocyte sedimentation rate after a little more than one month. The patient was discharged after one month and nine days. The response was excellent.

An investigation of immunological parameters in this patient revealed a marked increase in the lymphocyte stimulation test of peripheral blood.

## Clinical Example 2

The patient, a 82-year-old woman, had a past history of pulmonary tuberculous mycobacterial infection which developed formerly and was cured with antituberculosis drugs.

This case is recurrent infection, yielding many nontuberculous mycobacteria showing a negative nyacin test (Gaffky 2). The insolated bacteria were identified to be Mycobacterium avium-complex. The patient was treated by the administration of rifampicin 300 mg/day, ethambutol 0.4 g/day and isoniazid 150 mg/day through oral administration after admission in a hospital and continued for 3 months but no effect was achieved. Thereafter, concurrent intravenous administration of IL-2 in a dose of 1000 U/day was started as an adjunct to the above regimen. As a result, the bacteria ceased to be discharged and the patient became afebrile and showed improvement in pneumonia and erythrocyte sedimentation rate after a little more than ten days. The response was excellent.

An investigation of immunological parameters in this patient revealed a marked increase in the lymphocyte stimulation test of peripheral blood and the tuberculin test by the administration of said medicines.

## Clinical Example 3

The patient, a 66-year-old woman, had a past history of tuberculous mycobacterial infection which developed at the age of about 64. The patient is febrile of 38.5°C, and cough and phlegm are very increased so that the patient was admitted in a hospital. This case yields many acid-fact bacteria showing a negative nyacin test (Gaffky 3). The isolated bacteria were identified to be Mycobacterium avium-complex and were resistant to rifampicin and resistant to ethambutol and isoniazid. The patient was treated by the administration of rifampicin 300 mg/day, ethambutol 0.5 g/day and isoniazid 300 mg/day through oral administration after admission in a hospital and continued for 4 months but no effect was achieved. Thereafter, concurrent intravenous administration of IL-2 in a dose of 1000 U/day was started as an adjunct to the above regimen for 5 months. As a result, the bacteria ceased to be discharged and the patient became afebrile and showed improvement in number of peripheral blood lymphocytes. The patient does not feel weary of the whole body and become well. The response was good.

An investigation of immunological parameters in this patient revealed a marked increase in the lymphocyte stimulation test of peripheral blood and in tuberculin test.

## Clinical Example 4

The patient, a 33-year-old man, had a past history of pulmonary tuberculous mycobacterial infection which developed at the age of 18. At the age of 18 and 27, the patient was admitted in a hospital for 1 and half year respectively, but the isolated bacteria were resistant to rifampicin and the patient was not completely cured.

At this admission in a hospital, there are found many large cavities in the left and right lungs of the patient, many number of bacteria are discharged, and the patient is enervated. This case is a serious case of pulmonary tuberculous mycobacterial infection.

The isolated bacteria were identified to be Mycobacterium tuberculosis, and were sensitive to streptomycin, but resistant to isoniazid, ethanbutol and rifampicin. The administration of streptomycin 0.75 g/day was instituted and continued for 100 days, but any improvement was not found. After that, the oral administration of thionamid 0.4 g/day and rinderon 4 mg/day in addition to streptomycin 0.75 g/day, but any improvement was not found.

Thereafter, concurrent intravenous administration of IL-2 in a dose of 1000 U/day was started as an adjunct to the above regimen starting from 141 days after the streptomycin administration.

As a result, the condition of the whole body of the patient was improved, the patient became well and showed improvement in C reactive protein (CRP) and in erythrocyte sedimentation rate.

An investigation of immunological parameters in this patient revealed a marked increase in the lymphocyte stimulation test of peripheral blood and in tuberculin test.


### Example 1


In distilled water for injection were dissolved 23 mg/ml of aminoacetic acid, 0.11 ml/ml of 0.1 N-hydrochloric acid, 5 mg/ml of human serum albumin and 650 U/ml of human IL-2. The resulting aqueous solution at pH 3.9 was passed through a bacterial filter, distributed in 1 ml portions into vials, and frozen at -40°C. After drying, the vials were purged with $N_2$ gas, capped and pinched to provide an injectable product.


### Example 2


In distilled water for injection were dissolved 50 mg/ml of mannitol, 0.05 ml/ml of 0.1 N-hydrochloric acid, 5 mg/ml of human serum albumin and 650 U/ml of human IL-2. The resulting aqueous solution at pH 3.4 was filtered through a bacterial filter, distributed in 1 ml portions into vials, and frozen at -40°C. After drying, the vials were purged with $N_2$ gas, capped and pinched to provide an injectable product.


### Example 3

In distilled water for injection were dissolved 15 mg/ml of sorbitol, 35 mg/ml of mannitol, 0.035 ml/ml of 0.1 N-hydrochloric acid, 5 mg/ml of human serum albumin and 650 U/ml of human IL-2. The resulting aqueous solution at pH 3.7 was filtered through a bacterial filter, distributed in 1 ml portions in vials, and frozen at -40°C. After drying, the vials were purged with $N_2$ gas, capped and pinched to provide an injectable product.


## Claims

1. Use of an interleukin-2 active substance for the production of a pharmaceutical preparation for treating a tuberculous and nontuberculous mycobacterial infection in a human patient.

2. Use as claimed in Claim 1, wherein the preparation is for treating nontuberculous mycobacterial infection in a human patient.

3. Use as claimed in Claim 2, wherein the infection is caused by Mycobacterium avium-complex.

4. Use as claimed in Claim 1, wherein the preparation is for treating tuberculous mycobacterial infection in a human patient.

5. Use as claimed in Claim 1, wherein the interleukin2 active substance is a recombinant non-glycosylated human interleukin-2.

6. Use as claimed in Claim 1, wherein the interleukin2 active substance does not have a methionine residue at the amino terminus.

7. Use as claimed in Claim 1, wherein the preparation is in the form of injection.

Fig. 1

Ala Pro Thr Ser Ser Ser Thr Lys Lys Thr Gln Leu Gln

Leu Glu His Leu Leu Leu Asp Leu Gln Met Ile Leu Asn

Gly Ile Asn Asn Tyr Lys Asn Pro Lys Leu Thr Arg Met

Leu Thr Phe Lys Phe Tyr Met Pro Lys Lys Ala Thr Glu

Leu Lys His Leu Gln Cys Leu Glu Glu Glu Leu Lys Pro

Leu Glu Glu Val Leu Asn Leu Ala Gln Ser Lys Asn Phe

His Leu Arg Pro Arg Asp Leu Ile Ser Asn Ile Asn Val

Ile Val Leu Glu Leu Lys Gly Ser Glu Thr Thr Phe Met

Cys Glu Tyr Ala Asp Glu Thr Ala Thr Ile Val Glu Phe

Leu Asn Arg Trp Ile Thr Phe Cys Gln Ser Ile Ile Ser

Thr Leu Thr